# EUROPEAN PATENT APPLICATION

(11) **EP 3 591 658 A1**
(43) Date of publication of application: **08.01.2020**
(21) Application number: 18181623.2
(22) Date of filing: 04.07.2018
(51) Int. Cl.: G16H 20/30, G16H 40/63

(54) **METHOD AND APPARATUS FOR HAND FUNCTION MONITORING**

(71) Applicant: Nokia Technologies Oy, 02610 Espoo (FI)
(72) Inventor: Rajala, Satu, 36200 Kangasala (FI); Müller, Kiti, 00430 Helsinki (FI)
(74) Representative: Espatent Oy

(57) **Abstract**

An apparatus for monitoring hand function, comprising means for measuring a force applied by a finger of a user gripping the apparatus; and means for providing instructions to the user relating to applying force to the means for measuring a force; and for analyzing the force measured by the means for measuring a force in order to analyze the hand function.

## Description

### TECHNICAL FIELD

The present application generally relates to a method and apparatus for monitoring of force and movement. In particular, but not exclusively, the present application relates to a method and apparatus for monitoring human hand function.

### BACKGROUND

This section illustrates useful background information without admission of any technique described herein representative of the state of the art.

Functions, i.e. movement and strength, of human hand and wrist are the outcome of integrating together motor and sensory nerves and muscles as well as joint movements of the upper arm. These functions are primarily generated by the brain sensorimotor neural networks of the parietal lobes. Producing and controlling different finger, hand and wrist functions is the outcome of a sensorimotor feedback loop: Information of the positions of fingers, hand and wrist and tactile information on things a hand is handling are forwarded via different sensory nerves to the somatosensory cortex.

Typically, examination of upper arm motor and sensory nerve and muscle functions is done by asking the subject to perform certain motor functions with the hand and wrist that are known to represent especially the function of certain nerves and muscles. Such an examination is always subject to interpretation of the examiner and difficult to carry out in a repeatable manner.

Known apparatuses for assessing, or training, hand functions include simple handgrip strength measurements, and devices meant for exercising handgrip or finger strength. Such devices are of little use in monitoring complex hand functions. Furthermore, neurophysiological methods, such as electroneuromyography, are used in assessing related neural functions.

In view of the above, there is a need for a simple method and apparatus for assessing hand functions objectively and in a repeatable manner.

### SUMMARY

Various aspects of examples of the invention are set out in the claims.

According to a first example aspect of the present invention, there is provided an apparatus for monitoring hand function, comprising
means for measuring a force applied by a finger of a user gripping the apparatus; and
means for providing instructions to the user relating to applying force to the means for measuring a force; and for
analyzing the force measured by the means for measuring a force in order to analyze the hand function.

The means for measuring a force may comprise at least one force sensitive element.

The at least one force sensitive element may be configured to have force applied thereto by a specific finger of a human hand, respectively.

The means for measuring a force may comprise at least one actuator for providing stimuli to the user of the apparatus and providing instructions may comprise providing a stimulus.

The stimuli may comprise haptic, tactile or optical stimuli.

The means for providing instructions may comprise an audio device and/or a user interface element and providing instructions may comprise audio and/or visual instructions.

The instructions may comprise instructions as to which force sensitive elements force is to be applied by a finger.

The apparatus may further comprise means for measuring movement of the apparatus and therethrough the movement of the hand gripping the apparatus.

The instructions may comprise instructions as to how to move the hand gripping the apparatus.

The apparatus may comprise a handheld electronic device.

According to a second example aspect of the present invention there is provided a method for monitoring hand function, comprising
providing instructions to the user relating to applying force to means for measuring a force comprised in an apparatus gripped by the user;
measuring the force applied to the means for measuring a force by a finger of the user; and
analyzing the force measured by the means for measuring a force in order to analyze the hand function.

Measuring the force applied to the means for measuring a force by a finger of the user may comprise measuring force applied to at least one force sensitive element.

Each force sensitive element may be configured to have force applied thereto by a specific finger of a human hand, respectively.

Providing instructions to the user relating to applying force to the at least one force sensitive element may comprise providing haptic, tactile or optical stimuli to the user.

Providing instructions to the user relating to applying force to the at least one force sensitive element may comprise providing audio and/or visual instructions to the user.

The instructions may comprise instructions as to which force sensitive elements force is to be applied by a finger.

The method may further comprise measuring movement of the apparatus and therethrough the movement of the hand gripping the apparatus.

The instructions may comprise instructions as to how to move the hand gripping the apparatus.

According to a third example aspect of the present invention, there is provided an apparatus according to the first example aspect of the present invention, wherein the processor is configured to cause carrying out the method of the second example aspect of the present invention.

According to a fourth example aspect of the present invention, there is provided a computer program comprising code for causing carrying out the method of the second example aspect of the present invention when the computer program is run on a processor.

The computer program may be a computer program product comprising a computer-readable medium bearing computer program code embodied therein for use with a computer

Any foregoing memory medium may comprise a digital data storage such as a data disc or diskette, optical storage, magnetic storage, holographic storage, opto-magnetic storage, phase-change memory, resistive random access memory, magnetic random access memory, solid-electrolyte memory, ferroelectric random access memory, organic memory or polymer memory. The memory medium may be formed into a device without other substantial functions than storing memory or it may be formed as part of a device with other functions, including but not limited to a memory of a computer, a chip set, and a sub assembly of an electronic device.

Different non-binding example aspects and embodiments of the present invention have been illustrated in the foregoing. The embodiments in the foregoing are used merely to explain selected aspects or steps that may be utilized in implementations of the present invention. Some embodiments may be presented only with reference to certain example aspects of the invention. It should be appreciated that corresponding embodiments may apply to other example aspects as well.

### BRIEF DESCRIPTION OF THE DRAWINGS

For a more complete understanding of example embodiments of the present invention, reference is now made to the following descriptions taken in connection with the accompanying drawings in which:
Fig. 1A shows a schematic front view of an apparatus according to an example embodiment;
Fig. 1B shows a schematic front view of an apparatus according to an example embodiment;
Fig. 1C shows a schematic front view of an apparatus according to an example embodiment;
Fig. 1D shows a schematic front view of an apparatus according to an example embodiment;
Fig. 1E shows a schematic front view of an apparatus according to an example embodiment;
Fig. 2A shows a schematic principle view of operation of an apparatus according to an example embodiment;
Fig. 2B shows a schematic principle view of operation of an apparatus according to an example embodiment;
Fig. 2C shows a schematic principle view of operation of an apparatus according to an example embodiment;
Fig. 2D shows a schematic principle view of operation of an apparatus according to an example embodiment;
Fig. 3 shows a block diagram of a an apparatus according to an example embodiment; and
Fig. 4 shows a flow chart of a method according to an example embodiment.

### DETAILED DESCRIPTON OF THE DRAWINGS

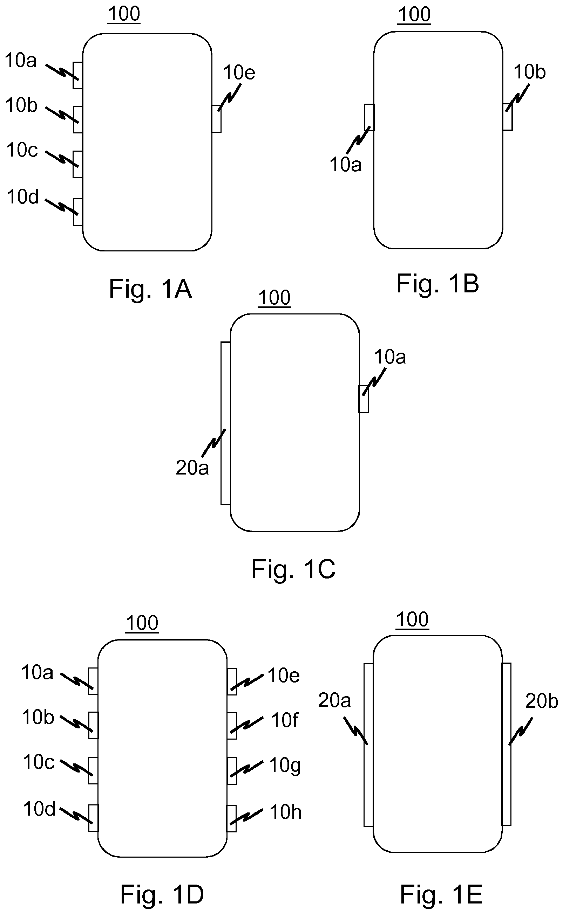

An example embodiment of the present invention and its potential advantages are understood by referring to Figs. 1A through 4 of the drawings. In this document, like reference signs denote like parts or steps.

Fig. 1A shows a schematic front view of an apparatus for hand function monitoring according to an example embodiment. The apparatus 100 is configured to be gripped by a user, i.e. by a human hand. The apparatus 100 comprises means for measuring a force applied thereto. In an example embodiment, the means for measuring a force comprise at least one force sensitive element 10a-e configured to measure force applied thereto. In the example embodiment of Fig. 1A the apparatus 100 comprises five force sensitive elements 10a-e, four on a first side of the apparatus 100 and one on the second, opposite side of the apparatus. In an example embodiment, the five force sensitive elements 10a-e correspond to the fingers of a human hand, the force sensitive element 10e alone on the second side corresponding to the thumb, i.e. each force sensitive element 10a-e is configured to have force applied thereto by a finger.

The term hand function used hereinbefore and hereinafter refers to any function performed by the human fingers, hand, wrist and/or arm. Hand functions are the outcome of integrating together motor and sensory nerves and muscles as well as joint movements of the upper arm. These functions are primarily generated by the brain sensorimotor neural networks of the parietal lobes. Producing and controlling different hand functions is the outcome of a sensorimotor feedback loop. Information of the positions of fingers, hand and wrist and tactile information on things a hand is handling are forwarded via different sensory nerves to the somatosensory cortex. This input of sensory information is primarily handled by neural networks of the motor cortex and thus sensory input influences motor functions. The motor nerves originating from the motor cortex innervate the different muscles of the human body. The performance of hand functions is indicative of the functioning of the parts involved in their production and control.

Fig. 1B shows a schematic front view of an apparatus for hand function monitoring according to an example embodiment. The apparatus 100 is configured to be gripped by a user, i.e. by a human hand. The apparatus 100 comprises means for measuring a force applied thereto. In an example embodiment, the means for measuring a force comprise at least one force sensitive element 10a-b configured to measure force applied thereto. In the example embodiment of Fig. 1B the apparatus 100 comprises two force sensitive elements 10a-b, one on each side of the apparatus 100 and one on the second, opposite side of the apparatus. In an example embodiment, the force sensitive element 10a on the first side of the apparatus is configured to be used, i.e. configured to have force applied thereto by a finger, by the other fingers of the hand alternately while the force sensitive element 10b alone on the second side corresponds to the thumb.

Fig. 1C shows a schematic front view of an apparatus for hand function monitoring according to an example embodiment. The apparatus 100 is configured to be gripped by a user, i.e. by a human hand. The apparatus 100 comprises means for measuring a force applied thereto. In an example embodiment, the means for measuring a force comprise at least one force sensitive element 10a, 20a configured to measure force applied thereto. In the example embodiment of Fig. 1C the apparatus 100 comprises two force sensitive elements 10a,20a, one force sensitive element 20a on a first side of the apparatus 100 and one force sensitive element 10a on the second, opposite, side of the apparatus. In an example embodiment, force sensitive element 20a on the first side of the apparatus has a width larger than the force sensitive element 10a alone on the second side corresponding to the thumb, so that the force sensitive element 20a is configured to be used for the other fingers of the hand, i.e. configured to have force applied thereto by a finger. In an example embodiment, the force sensitive element 20a comprises an array of force sensitive elements. In a further example embodiment, the array of force sensitive element comprises a force sensitive element assigned to each finger, or several force sensitive elements assigned to each finger.

Hereto it is noted that the example embodiment of the apparatus 100 depicted in Figs 1A-C is configured to be held in a right hand of the user. It is apparent that in an example embodiment, the force sensitive elements would be positioned on opposite side, respectively, in which case the apparatus 100 was a mirror image and configured to be held in a left hand of the user.

Fig. 1D shows a schematic front view of an apparatus for hand function monitoring according to an example embodiment. The apparatus 100 is configured to be gripped by a user, i.e. by a human hand. The apparatus 100 comprises means for measuring a force applied thereto. In an example embodiment, the means for measuring a force comprise at least one force sensitive element 10a-h configured to measure force applied thereto. In the example embodiment of Fig. 1D the apparatus 100 comprises eight force sensitive elements 10a-h, four on a first side of the apparatus 100 and four on the second, opposite, side of the apparatus. In an example embodiment, the eight force sensitive elements 10a-h are configured to be used by the fingers of either human hand, a force sensitive element on one side corresponding to the thumb and the force sensitive element on the other side of the apparatus configured to be used by the other fingers of the hand.

Fig. 1E shows a schematic front view of an apparatus for hand function monitoring according to an example embodiment. The apparatus 100 is configured to be gripped by a user, i.e. by a human hand. The apparatus 100 comprises means for measuring a force applied thereto. In an example embodiment, the means for measuring a force comprise at least one force sensitive element 20a-b configured to measure force applied thereto. In the example embodiment of Fig. 1E the apparatus 100 comprises two force sensitive elements 20a-b, one force sensitive element 20a on a first side of the apparatus 100 and one force sensitive element 20b on the second, opposite, side of the apparatus. In an example embodiment, the two force sensitive elements 20a-b are configured to be used by the fingers of either human hand, a force sensitive element 20a,b on one side corresponding to the thumb and the force sensitive element 20a,b on the other side of the apparatus configured to be used by the other fingers of the hand. In an example embodiment, the force sensitive element 20a comprises an array of force sensitive elements. In a further example embodiment, the array of force sensitive element comprises a force sensitive element assigned to each finger, or several force sensitive elements assigned to each finger.

Hereto it is noted that although Figs. 1A-1F depict certain example embodiments with the means for measuring a force comprising a certain number of force sensitive elements 10a-h,20a-b, the number and combination of force sensitive elements is not limited thereto. In an example embodiment, the apparatus comprises for example force sensitive elements meant to be used by two or three fingers, or force sensitive elements on both ends of the apparatus as well, or only a single force sensitive element to be used with each finger alternately. In a further example embodiment, the force sensitive elements are positioned only on a single side of the apparatus, for example on the front or on a side. In a still further embodiment, the apparatus 100 comprises force sensitive elements 10a-h, 20a-b in such an arrangement that the apparatus can be held and used with both hands simultaneously, thus providing the possibility to monitor the hand functions of both hands and their difference and/or synchronization as well as providing a cognitive element to hand function monitoring, since using both hands requires more brain level con-ordination.

In an example embodiment, the force sensitive elements 10a-h comprise force sensitive buttons. In an example embodiment, the buttons comprise hardware buttons or soft buttons or a combination thereof comprising. In an example embodiment, the force sensitive elements 20a,20b comprise a matrix, array of force sensors so that the grip locations on which the force applied by a finger is measured are not limited, i.e. the grip can change and the fingers can be located also very near to each other in case for example a child using the apparatus.. In an example embodiment, the force sensitive elements 10a-h,20a-b comprise a portion of a touch sensitive surface, for example of touch sensitive display, configured to be sensitive to force applied thereto. In a still further example, the whole surface of the apparatus 100, or a predetermined part of the surface of the apparatus, such as two opposite side surfaces, is configured to be sensitive to force applied thereto.

The force sensitive elements 10a-h,20a-b are configured to measure a force applied thereto. In an example embodiment, the force sensitive elements 10ah,20a-b are configured to measure an absolute force applied thereto. In an example embodiment, the force sensitive elements 10a-h,20a-b are configured to measure, in a a momentary force, a change in force and/or the duration of force being applied. In an example embodiment, the force sensitive elements 10a-h,20a-b comprise at least one force sensor selected from the group of strain gauge, piezoresistive sensor, piezoelectric sensor, capacitive sensor, optical sensor and magnetic sensor.

In an example embodiment, the apparatus 100 comprises further sensors or measurement means, not shown in Figs. 1A-E, in addition to the force sensitive elements 10a-h,20a-b. In an example embodiment, the apparatus 100 comprises means for measuring movement. In an example embodiment, the means for measuring movement comprise an accelerometer configured to measure acceleration and therethrough movement and vibration. In an example embodiment, the means for measuring movement comprise a gyroscope configured to measure the angular speed of the apparatus and therethrough the hand holding the apparatus, e.g. for determining movement and/or position thereof. In a further example embodiment, the means for measuring movement comprise a microelectromechanical system, MEMS, based combination sensor comprising in an example embodiment an accelerometer, a gyroscope and a magnetometer.

In an example embodiment, the accelerometer, the gyroscope, and/or a combination sensor are configured to measure movement and related quantities such as velocity, acceleration, vibration and position in order to monitor movements of the hand holding the device, such as wrist flexion, deviation, extension, pronation and/or supination. In a further example embodiment, the apparatus 100 is attached to a limb of a human, for example to an arm, a leg, or a foot, and the accelerometer, the gyroscope, and/or a combination sensor are configured to measure movement and related quantities such as velocity, acceleration, vibration and position in order to monitor movements of the limb which the apparatus 100 is attached to.

In an example embodiment, the apparatus 100 comprises a personal electronic device such as a mobile phone, a smartphone, a gaming device, a tablet computer, a smartwatch, an e-book reader, a dedicated electronic device, or a part of a larger device that can be gripped by a human hand.

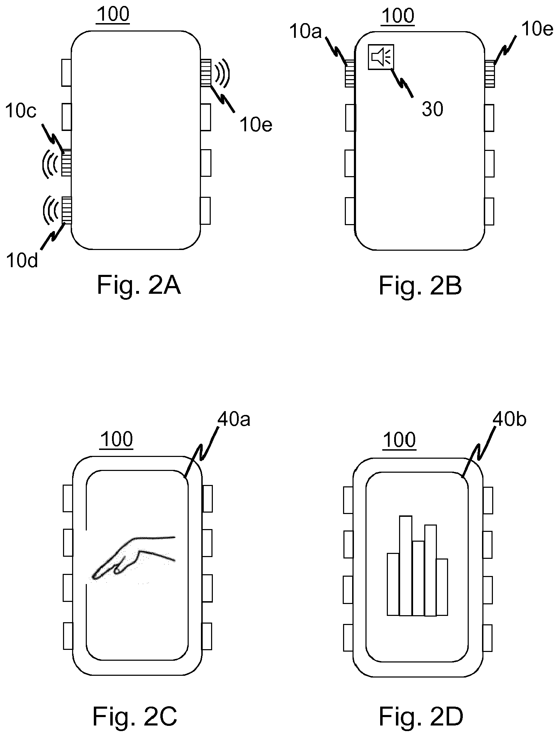

Fig. 2A shows a schematic principle view of operation of an apparatus according to an example embodiment. The apparatus 100 depicted in Figs. 2A-2D corresponds to an apparatus 100 according to the embodiments explained hereinbefore with reference to Figs 1A-1E. In the example embodiment of Fig. 2A, the user of the apparatus 100A is supposed to apply force to force sensitive elements 10c-e. In order to instruct the user to apply force to the desired force sensitive elements 10c-e, the user is in an example embodiment given a haptic stimulus, i.e. a stimulus relating to or based on the sense of touch, or a tactile stimulus, i.e. a stimulus perceptible by touch. In an example embodiment, the force sensitive elements to which the force is to be applied, i.e. instructing the user with which fingers the user should grip and/or pinch, vibrate. In a further example embodiment, the stimulus is given optically, i.e. the force sensitive elements to which the force is to be applied, i.e. instructing the user with which fingers the user should grip and/or pinch, are illuminated. In a further example embodiment, the stimulus is given with temperature, i.e. the temperature of the force sensitive elements to which the force is to be applied, i.e. instructing the user with which fingers the user should grip and/or pinch, is changed. In an embodiment, the means for measuring a force, in an embodiment the force sensitive elements 10a-h,20a-b, comprise at least one actuator configured to provide the stimulus, for a led light or a vibrator, such as a piezoelectric vibrator.

Fig. 2B shows a schematic principle view of operation of an apparatus according to an example embodiment. In the example embodiment of Fig. 2B, the user of the apparatus 100A is supposed to apply force to force sensitive elements 10a, 10e. In order to instruct the user to apply force to the desired force sensitive elements 10c-e, the user is in an example embodiment given instructions, instead of or in addition to other stimuli, by using means for providing instructions, for example by using an audio device 30 comprised in the apparatus, i.e. audio recording instructing the user with which fingers the user should grip and/or pinch, is played.

Fig. 2C shows a schematic principle view of operation of an apparatus according to an example embodiment. In the example embodiment of Fig. 2B, the user of the apparatus 100A is supposed to move the hand holding the device in a certain manner, for example to flex the wrist. In an example embodiment, the apparatus 100 comprises means for providing instructions to the user. In an example embodiment, the means for providing instructions comprise a user interface element 40a configured to show visual instructions, for example a video, animation or and image, for the user to carry out the required movement. In an example embodiment also instruction concerning applying force to force sensitive elements 10a-h,20a-b are provided as visual instruction on the user interface element 40a. In an example embodiment, the user interface element 40a comprises a display device. In an example embodiment, the display device comprises a touch sensitive display. In a further example embodiment, some or all of the force sensitive elements 10a-h, 20a-b are comprised in the user interface element 40a.

Fig. 2D shows a schematic principle view of operation of an apparatus according to an example embodiment. In the example embodiment of Fig. 2D, the user of the apparatus has carried out the instructed movements, pinches and /or grips. In an example embodiment, the apparatus 100 comprises means for showing a result of or an analysis of the monitoring of hand function, for example a user interface element 40b configured to show, for example as visual elements, as depicted in Fig. 2D as a bar graph, a result or an analysis of the monitoring of hand function. In an example embodiment, the user interface element 40b comprises a display device. In an example embodiment, the user interface element 40b is the same user interface element or integrated with the user interface element 40a. In an example embodiment, the display device comprises a touch sensitive display. In a further example embodiment, some or all of the force sensitive elements 10a-h, 20a-b are comprised in the user interface element 40b.

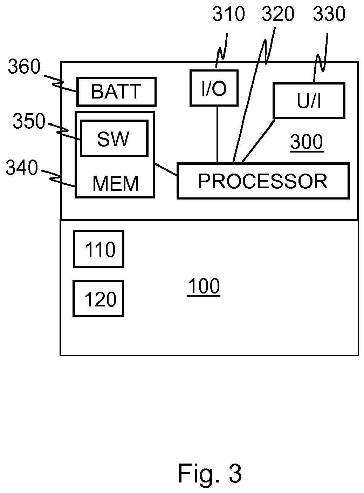

Fig. 3 shows a block diagram of the apparatus 100 and a control element 300 according to an example embodiment of the invention. Fig. 3 further shows sensor elements 110 and 120 comprised in the apparatus 100, comprising for example an accelerometer and a gyroscope as hereinbefore explained.

In an example embodiment, the control element 300 is an integral part of the apparatus 100, although it has been shown as separate block in Fig. 3 for intelligibility. In a further example embodiment, the control element is in part or wholly situated outside the apparatus 100, for example in a separate device or in a cloud service, in which case the apparatus 100 has integrated therewith the elements needed to communicate with the control element 300. The control element 300 comprises a memory 340 including a persistent computer program code 350. The control element 300 further comprises a processor 320 for controlling the operation of the apparatus 100 using the computer program code 350 and for causing carrying out the functions of the apparatus and the methods according to example embodiments. The control element further comprises a communication unit 310 for communicating with other apparatuses and/or with the elements of the apparatus 100, and in an example embodiment a user interface unit 330, in addition to or instead of the user interface element 40a,40b. In an example embodiment, the user interface unit comprises, on the outer surface of the apparatus 100 elements for controlling the functions of the apparatus 100. The control element, in an embodiment, further comprises a power source 360, such as a battery or a connection to an external power source.

In an example embodiment, the communication unit 310 is configured to provide communication with an external apparatus such as a smartphone, a smartwatch, a tablet computer, a laptop computer, a desktop computer, laboratory equipment, or a cloud service. The communication unit 310 comprises, for example, a local area network (LAN) port; a wireless local area network (WLAN) unit; Bluetooth unit; cellular data communication unit; or satellite data communication unit.

The processor 320 comprises, for example, any one or more of: a master control unit (MCU); a microprocessor; a digital signal processor (DSP); an application specific integrated circuit (ASIC); a field programmable gate array; and a microcontroller.

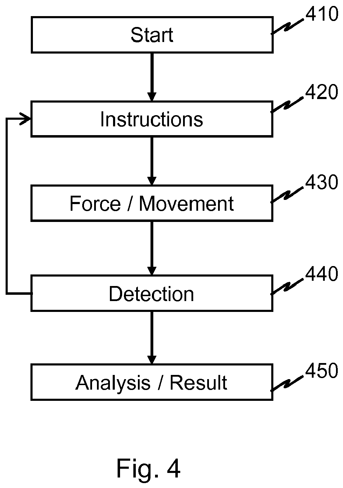

Fig. 4 shows a flow chart of a method according to an example embodiment. At step 410 the method for monitoring hand functions is started and the user, i.e. the person whose hand function is to be monitored takes the device 100 to hand and grips it with one hand or both hands.

At step 420 instructions are provided for the user as to what should be done, i.e. which force sensitive elements should force be applied to and/or what kind of movements should be carried out. In an example embodiment the instructions are given as hereinbefore described with haptic, tactile, audio and/or visual stimuli. In further example embodiment, the instructions are be received from an external source, for example from an external apparatus, or from a person assisting in the monitoring.

At step 430, the user of the apparatus carries out, to the best of his/her ability, the instructed movements, pinches and grips, i.e. moves the hand in the instructed manner and/or applies force to the force sensitive elements 10a-h,20a-b as instructed. At step 440, the applied force and the movement is detected and measured and the result is stored into the memory 340 or sent to an external device for storing.

In an example embodiment, the monitoring of hand function requires several different movements and grips, i.e. tests, to be carried out and accordingly the steps 420 to 440 are repeated until all the necessary movements and grips have been carried out. In an example embodiment, a predetermined sequence, stored into the apparatus 100 is carried out. In a further example embodiment, the number and type of tests depends on the results of the previous tests and the sequence of instructions is adapted in accordance therewith.

At step 450 a result of the monitoring of hand function is analyzed, i.e. a result is calculated based on the measured force and movement data, and the result is provided. In an example embodiment, the analysis is carried out using means for analyzing the force measured by the means for measuring a force in order to analyze the hand function. In an example embodiment, the means for analyzing comprise the processor 320. In an example embodiment, the result is shown to the user on a user interface element 40b and/or sent to an external device for further analysis or review. In a further example embodiment, the result is compared with previously stored results in order to assess change and/or improvement. In a further example embodiment, the apparatus 100 is configured to provide an assessment of possible condition or fault in the hand function based on the result.

Without in any way limiting the scope, interpretation, or application of the claims appearing below, a technical effect of one or more of the example embodiments disclosed herein is the provision of a repeatable examination and monitoring of hand function. Another technical effect of one or more of the example embodiments disclosed herein is the provision of an easy to use monitoring device for hand function monitoring and training. Another technical effect of one or more of the example embodiments disclosed herein is the possibility for reliably carrying out different types of tests. Another technical effect of one or more of the example embodiments disclosed herein is an improved possibility for long-time monitoring.

Some example use cases of the method and apparatus for hand function monitoring are presented in the following. In a first use case the method and apparatus for hand function monitoring is used at a clinical examination of hand function, for example by a medical professional. In such a case a predetermined sequence of tests is carried out and a diagnosis can be formed based on the analysis of the result. The test sequence depends on the specific hand function, and the underlying cause to be tested, for example when testing the function of ulnar nerve, the pinch strength of the 4th and 5th finger are tested.

In a second use case the method and apparatus for hand function monitoring is used in rehabilitation for example after an injury or operation. In such a case a sequence of tests is carried out by the user, for example a home, at predetermined times, regularly, and the progress and is monitored from the results and the rehabilitation program is adjusted as needed based thereon.

In a third use case the method and apparatus for hand function monitoring is used in training finger strength and movements for example by a child or a musician, such as a guitarist. In such a case a sequence of instructions aimed at improving strength or fingering can be designed by the user and progress monitored regularly.

In a fourth use case the method and apparatus for hand function monitoring is used as an aide during examination of the brain, for example electroencephalography, EEG, for activating certain areas of the brain based on a test sequence designed for that purpose.

In fifth use case the method and apparatus for hand function monitoring is used in assessing the effects of stress and/or fatigue on hand function by carrying out a certain test sequence at different stages of stress and fatigue.

In a sixth use case the method and apparatus for hand function monitoring is used to assess sensitivity to different stimuli, i.e. a test sequence requiring to carry out same tests but instructed with different stimuli is carried out and the results are compared.

Embodiments of the present invention may be implemented in software, hardware, application logic or a combination of software, hardware and application logic. The software, application logic and/or hardware may reside on the apparatus 100. If desired, part of the software, application logic and/or hardware may reside on the apparatus 100, part of the software, application logic and/or hardware may reside on an external device, and/or part of the software, application logic and/or hardware may reside on a cloud service. In an example embodiment, the application logic, software or an instruction set is maintained on any one of various conventional computer-readable media. In the context of this document, a "computer-readable medium" may be any non-transitory media or means that can contain, store, communicate, propagate or transport the instructions for use by or in connection with an instruction execution system, apparatus, or device, such as a computer, with one example of a computer described and depicted in Fig. 3. A computer-readable medium may comprise a computer-readable storage medium that may be any media or means that can contain or store the instructions for use by or in connection with an instruction execution system, apparatus, or device, such as a computer.

If desired, the different functions discussed herein may be performed in a different order and/or concurrently with each other. Furthermore, if desired, one or more of the before-described functions may be optional or may be combined.

Although various aspects of the invention are set out in the independent claims, other aspects of the invention comprise other combinations of features from the described embodiments and/or the dependent claims with the features of the independent claims, and not solely the combinations explicitly set out in the claims.

It is also noted herein that while the foregoing describes example embodiments of the invention, these descriptions should not be viewed in a limiting sense. Rather, there are several variations and modifications which may be made without departing from the scope of the present invention as defined in the appended claims.

## Claims

1. An apparatus for monitoring hand function, comprising
means for measuring a force applied by a finger of a user gripping the apparatus; and
means for
providing instructions to the user relating to applying force to the means for measuring a force; and for
analyzing the force measured by the means for measuring a force in order to analyze the hand function.

2. The apparatus of claim 1, wherein the means for measuring a force comprise at least one force sensitive element.

3. The apparatus of any preceding claim, wherein the means for measuring a force comprise at least one actuator for providing stimuli to the user of the apparatus and providing instructions comprises providing a haptic, tactile or optical stimulus.

4. The apparatus of any preceding claim, wherein the means for providing instructions comprise an audio device and/or a user interface element and providing instructions comprises audio and/or visual instructions.

5. The apparatus of any preceding claim 2-4, wherein the instructions comprise instructions as to which force sensitive elements force is to be applied by a finger.

6. The apparatus of any preceding claim, further comprising means for measuring movement of the apparatus and therethrough the movement of the hand gripping the apparatus.

7. The apparatus of claim 7, wherein the instructions comprise instructions as to how to move the hand gripping the apparatus.

8. A method for monitoring hand function, comprising
providing instructions to the user relating to applying force to means for measuring a force comprised in an apparatus gripped by the user;
measuring the force applied to the means for measuring a force by a finger of the user; and
analyzing the force measured by the means for measuring a force in order to analyze the hand function.

9. The method of claim 8, wherein measuring the force applied to the means for measuring a force by a finger of the user comprises measuring force applied to at least one force sensitive element.

10. The method of any of the claims 8 to 9, wherein providing instructions to the user relating to applying force to the at least one force sensitive element comprises providing haptic, tactile or optical stimuli to the user.

11. The method of any of the claims 8 to 10, wherein the instructions comprise instructions as to which force sensitive elements force is to be applied by a finger.

12. The method of any of the claims 8 to 11, further comprising measuring movement of the apparatus and therethrough the movement of the hand gripping the apparatus.

13. The method of any of the claims 8 to 12, wherein the instructions comprise instructions as to how to move the hand gripping the apparatus.

14. A computer program, comprising code for causing carrying out the method of any of the claims 8 to 13 when the computer program is run on a processor.

15. The computer program according to claim 14, wherein the computer program is a computer program product comprising a computer-readable medium bearing computer program code embodied therein for use with a computer.
